# DEMANDE DE BREVET EUROPEEN

(11) **EP 1 161 935 A2**
(43) Date de publication de la demande: **12.12.2001**
(21) Numéro de dépôt: 01401197.7
(22) Date de dépôt: 10.05.2001
(51) Int. Cl.: A61K 7/06, A61K 7/48

(54) **Utilisation de particules de gel cubique comme agent anti-pollution, notamment dans une composition cosmétique**

(30) Priorité: 08.06.2000 FR 0007342
(71) Demandeur: L'OREAL, 75008 Paris (FR)
(72) Inventeur: Afriat, Isabelle, 75003 Paris (FR); Biatry, Bruno, 94300 Vincennes (FR)
(74) Mandataire: Rasson, Catherine

(57) **Abrégé**

La présente demande se rapporte à l'utilisation en application topique, de particules de gel cubique comme agent anti-pollution, notamment comme agent cosmétique anti-pollution.

La demande se rapporte aussi à un procédé de traitement cosmétique en vue de protéger l'organisme contre les effets de la pollution, consistant à appliquer sur la matière kératinique une composition contenant dans un milieu physiologiquement acceptable, une quantité efficace de particules de gel cubique.

Les particules de gel cubique sont de préférence en dispersion aqueuse et sont formées soit à partir d'un composé choisi parmi le 3,7,11,15-tétraméthyl-1,2,3-hexadecanetriol ou phytantriol, les dérivés N-2-alcoxycarbonyles de N-méthylglucamine et les monoglycérides d'acide gras insaturé, et d'un agent dispersant et stabilisant, soit par mélange d'au moins deux composés amphiphiles, l'un des composés amphiphiles étant susceptible de former en présence d'eau une phase lamellaire et l'autre étant susceptible de former en présence d'eau une phase hexagonale inverse.

## Description

La présente demande se rapporte à l'utilisation de particules de gel cubique comme agent anti-pollution, et aussi à un procédé de traitement cosmétique en vue de protéger l'organisme contre les effets de la pollution, consistant à appliquer sur la matière kératinique, une composition contenant dans un milieu physiologiquement acceptable, une quantité efficace de particules de gel cubique.

Certains milieux urbains sont régulièrement soumis à des pics de pollution. L'individu dans son environnement quotidien et particulièrement en zone urbaine, peut être soumis à de multiples agressions au niveau des matières kératiniques, et notamment de la peau, du cuir chevelu et des cheveux, par différents polluants aériens.

Parmi les polluants pouvant exercer des effets délétères sur les matières kératiniques, les gaz toxiques tels que l'ozone, le monoxyde de carbone, les oxydes d'azote ou les oxydes de soufre, sont l'un des constituants majeurs. Il a été constaté que ces gaz toxiques favorisent la desquamation des matières kératiniques, les rendent sales et ternes. De même ces gaz entraînent une asphyxie cellulaire au niveau desdites matières kératiniques.

On sait par ailleurs que les métaux lourds (plomb, cadmium, mercure) sont des polluants atmosphériques dont les émissions ont notablement augmenté, notamment en milieu urbain ou industriel. Outre certains effets toxiques qui leur sont propres, les métaux lourds ont la propriété de diminuer l'activité des moyens de défense cellulaire contre les radicaux libres [voir par exemple R.S. Dwivedi, J. Toxicol. Cut. & Ocular Toxical. 6(3), 183-191 (1987)]. Ainsi, les métaux lourds aggravent les effets toxiques des polluants gazeux en diminuant l'efficacité des moyens naturels de défense, et provoquent une accélération du phénomène de vieillissement cellulaire. Ceci est vrai en particulier pour les matières kératiniques et notamment la peau, le cuir chevelu et les cheveux qui sont en contact direct et permanent avec le milieu extérieur.

Ainsi, les effets nocifs de la pollution sur les matières kératiniques affectent la respiration cellulaire et se traduisent par un vieillissement accéléré de la peau, avec un teint terne et la formation précoce de rides ou ridules, et aussi par une diminution de la vigueur des cheveux qui prennent aussi un aspect terne. En outre, du fait de la pollution, peau et cheveux se salissent plus rapidement. De plus, la pollution peut provoquer des phénomènes d'allergie sur la peau.

Aussi, il existe le besoin de compositions permettant d'éviter les effets néfastes dus aux polluants (gaz ou métaux lourds), de façon à protéger les matières kératiniques de ces polluants externes.

Il a été maintenant constaté de manière tout à fait surprenante que les particules de gel cubique en application sur les matières kératiniques, et en particulier sur la peau, permettaient de protéger ces matières kératiniques, des effets des polluants se trouvant dans l'atmosphère.

Ainsi, la présente invention a pour objet l'utilisation cosmétique de particules de gel cubique comme agent anti-pollution dans une composition pour application topique sur les matières kératiniques.

L'invention a encore pour objet l'utilisation de particules de gel cubique pour la préparation d'une composition à application topique destinée à protéger la matière kératinique contre les effets nocifs de la pollution.

On entend ici par « application topique » une application externe sur les matières kératiniques, que sont notamment la peau, le cuir chevelu, les cils, les sourcils, les ongles et les muqueuses.

Les particules de gel cubique peuvent être utilisées seules et constituer la composition à utiliser, ou être incorporées dans une composition et notamment dans une émulsion huile-dans-eau (H/E) ou eau-dans-huile (E/H).

Les particules de gel cubique utilisées selon l'invention peuvent être présentes dans la composition pour application topique, en une quantité allant par exemple de 0,1 à 20 % en poids et de préférence de 0,1 à 10 % en poids par rapport au poids total de la composition.

Le terme de gel cubique désigne des gels transparents, isotropes en lumière polarisée, se présentant sous forme de phase cristal liquide cubique. Les phases cubiques sont organisées d'une manière bi-polaire en domaines hydrophile et lipophile distincts, en contact étroit et formant un réseau tridimensionnel thermodynamiquement stable. Une telle organisation a été notamment décrite dans Luzzati (1968), « Biological Membranes » (Chapman, D. Ed.), vol 1, 71-123 et dans Mariani et coll. (1988), J. Mol. Biol., 204, 165-189, ainsi que dans "La Recherche" (1992), Vol.23, 306-315. Selon l'arrangement des domaines hydrophile et lipophile, la phase cubique est dite de type normal ou inverse. Le terme de gel cubique utilisé selon la présente invention regroupe bien entendu les gels présentant les différents types de phases cubiques.

Quand le gel cubique est dispersé dans un milieu aqueux, on obtient des particules de gel cubique en dispersion, particules qui ont la même structure que le gel cubique non dispersé.

Tous les types de particules de gel cubique peuvent être utilisés selon la présente invention. De manière avantageuse, les particules de gel cubique utilisées sont en dispersion aqueuse. Elles peuvent être notamment obtenues selon les deux modes préférés de réalisation décrits ci-dessous.

Selon un premier mode de réalisation, les particules se trouvent en dispersion aqueuse et sont formées par un mélange comprenant (i) de 0,1 à 15 % en poids par rapport au poids total de la composition, d'au moins un composé choisi parmi le 3,7,11,15-tétraméthyl-1,2,3-hexadecanetriol ou phytantriol, les dérivés N-2-alcoxycarbonyles de N-méthylglucamine et les monoglycérides d'acide gras insaturé, et (ii) de 0,05 à 3 % en poids par rapport au poids total de la composition, d'au moins un agent dispersant et stabilisant, ledit agent étant choisi parmi les agents tensioactifs hydrosolubles à température ambiante, contenant une chaîne grasse, saturée ou insaturée, linéaire ou ramifiée, ayant de 8 à 22 atomes de carbone. Selon ce mode de réalisation des particules de gel cubique utilisées selon l'invention, la proportion pondérale relative en composé (i) par rapport au poids à l'agent dispersant et stabilisant (ii) peut aller par exemple de 2 à 200, et de préférence de 5 à 50.

Le phytantriol utilisable comme composé (i) est un composé connu, notamment commercialisé sous la dénomination de « Phytantriol-63926 » par la Société Roche.

Parmi les dérivés N-2-alcoxycarbonyles de N-méthylglucamine, on peut notamment citer ceux répondant à la formule (I) suivante : 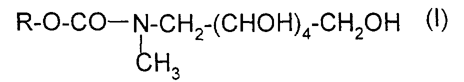 dans laquelle R représente un radical alkyle ramifié comportant de 6 à 18 atomes de carbone.

Parmi ceux-ci, on peut notamment mentionner la N-2-hexyldécyloxycarbonyl-N-méthyl-glucamine, la N-2-éthylhexyloxycarbonyl-N-méthyl-glucamine, la N-2-butyloctyloxycarbonyl-N-méthyl-glucamine et leurs mélanges.

Les composés de formule (I) tels que définis ci-dessus sont décrits et peuvent être préparés selon le procédé décrit dans le document EP-A-711540 cité ici pour référence. Ce procédé comprend notamment les étapes consistant :
(a) à dissoudre dans un mélange d'eau et d'un solvant organique, de la N-méthyl-glucamine, le solvant pouvant être par exemple le tétrahydrofuranne,
(b) à disperser dans le mélange obtenu précédemment, du bicarbonate de sodium, en une proportion appropriée correspondant environ à quatre fois la proportion molaire de N-méthyl-glucamine,
(c) à introduire alors dans le mélange réactionnel obtenu, un chloroformiate d'alkyle, le radical alkyle étant en C6-C18, en une proportion appropriée, généralement équimolaire par rapport à celle de N-méthyl-glucamine, puis à laisser réagir le mélange, et
(d) à filtrer le mélange réactionnel obtenu après l'étape (c), à recueillir le résidu pâteux obtenu par filtration, puis à le dissoudre dans de l'acétone en vue de sa cristallisation à une température de l'ordre de 5°C. Après filtration, les cristaux du dérivé N-2-alcoxycarbonyle de N-méthylglucamine formé sont essorés et séchés sous vide.

Dans le cas où l'on utilise des composé de formule (I) comme composé (i), les particules de gel cubique utilisées selon l'invention contiennent de préférence un mélange d'un tel composé et de phytantriol, et plus précisément un mélange comprenant une quantité de phytantriol allant de 1 à 40 % en poids et mieux de 10 à 30 % en poids par rapport au poids du mélange, et une quantité de dérivé N-2-alcoxycarbonyle de N-méthylglucamine de formule (I) allant de 60 à 99 % en poids et mieux de 70 à 90 % en poids par rapport au poids du mélange.

Les monoglycérides d'acide gras insaturé pouvant être utilisés comme composés (i) dans ce premier mode de réalisation sont de préférence ceux ayant une chaîne grasse insaturée comportant de 16 à 22 atomes de carbone. Parmi ceux-ci, on peut notamment citer le monooléate de glycéryle ou monooléine et le monolinoléate de glycéryle ou monolinoléine. On peut bien entendu utiliser pour préparer les dispersions de particules de gel cubique, un mélange de monoglycérides tels que définis précédemment ainsi qu'un mélange de monoglycérides d'acide gras insaturé et de monoglycérides d'acide gras saturé, la proportion en monoglycérides d'acide gras saturé étant cependant de préférence inférieure à celle de monoglycérides d'acide gras insaturé.

Dans le cas où l'on utilise des monoglycérides d'acide gras insaturé comme composé (i), les particules de gel cubique contiennent de préférence, comme composé (i), un mélange d'un tel composé et de phytantriol, et plus précisément un mélange comprenant une quantité de phytantriol allant de 1 à 50 % en poids et mieux de 10 à 30 % en poids par rapport au poids total du mélange et une quantité de monoglycéride d'acide gras insaturé allant de 50 à 99 % en poids et mieux de 70 à 90 % en poids par rapport au poids du mélange.

L'agent dispersant et stabilisant (ii) de ce premier mode de réalisation des particules de gel cubique est de préférence choisi parmi :
(1) les alkyl ou alcényl éthers ou esters de polyol,
(2) les amino-acides N-acylés et leurs dérivés, et les peptides N-acylés par un radical alkyle ou alcényle et leurs sels,
(3) les alkyl ou alcényl éthers ou esters sulfates, leurs dérivés et leurs sels,
(4) les alkyl ou alcényl éthers ou esters gras polyoxyéthylénés,
(5) les acides alkyl ou alcényl carboxyliques polyoxyéthylénés et leurs sels,
(6) les N-alkyl ou alcényl bétaïnes,
(7) les alkyl ou alcényl triméthylammonium et leurs sels, et
(8) leurs mélanges.

Dans les composés ci-dessus énumérés, les radicaux alkyle et alcényle ont de 8 à 22 atomes de carbone et peuvent se présenter sous forme de mélanges.
(1) Comme alkyl ou alcényl éthers ou esters de polyol, on peut citer notamment :
   (a) les alkyl ou alcényl esters de sorbitan polyoxyéthylénés comportant au moins 20 motifs d'oxyde d'éthylène, tels que le palmitate de sorbitan 20 OE ou Polysorbate 40 commercialisé sous la dénomination de « Montanox 40 DF » par la Société Seppic, et le laurate de sorbitan 20 OE ou Polysorbate 20 commercialisé sous la dénomination de « Tween 20 » par la Société ICI,
   (b) les alkyl ou alcényl esters de polyglycérol comportant au moins 10 motifs dérivés du glycérol, oxyéthylénés ou non, tels que le polyglycéryl-10 laurate commercialisé sous la dénomination de « Decaglyn 1-L » par la Société Nikko Chemicals,
   (c) les alkyl ou alcényl éthers de polyglycérol, tels que le polyglycéryl-3 hydroxylauryléther commercialisé sous la dénomination de « Chimexane NF » par la Société Chimex, et
   (d) les alkyl ou alcényl éthers ou esters de mono ou polysaccharides, tels que ceux dérivant du glucose, du fructose, du galactose, du maltose ou du lactose et notamment les monoesters en 1- et 6- du D-fructose, du décylglucose et du décylpolyglucose.
(2) Comme amino-acides N-acylés et dérivés, et comme peptides N-acylés par un radical alkyle ou alcényle, et sels, on utilise de préférence ceux pour lesquels le radical alkyle ou alcényle comporte au moins 12 atomes de carbone.
   Par amino-acides, on entend selon l'invention les alpha-, bêta- ou gamma-aminoacides. Comme sels d'amino-acides N-acylés, on peut citer par exemple les sels de N-acylglutamate, tels que le cocoylglutamate de monosodium, le lauroylglutamate de monosodium, l'alcoyl C14-C20 glutamate de disodium (le radical alcoyle C14-C20 dérivant du suif hydrogéné), commercialisés respectivement sous les dénominations de « Acylglutamate CS-11 », de « Acylglutamate LS-11 » et de « Acylglutamate HS-21 » par la Société Ajinomoto. On peut encore citer les lysines N-acylées telles que la lauroyl-lysine commercialisée sous la dénomination de « Amihope LL » par la Société Ajinomoto. Les dérivés d'amino-acides N-acylés et leurs sels sont de préférence les sarcosinates N-acylés tels que le lauroyl-sarcosinate de sodium commercialisé sous la dénomination de « Oramix L30 » par la Société Seppic, le myristoylsarcosinate de sodium et le palmitoylsarcosinate de sodium commercialisés respectivement sous les dénominations de « Nikkol Sarcosinate MN » et de « Nikkol Sarcosinate PN » par la Société Nikko Chemicals.
   Parmi les peptides N-acylés, on peut citer ceux dérivés de tout ou partie du collagène ou de la kératine tels que le lauroyl collagène de sodium et la palmitoyl kératine commercialisés sous les dénominations de « Proteol B 30 » et de « Lipacide PK » par la Société Seppic.
(3) Parmi les alkyl ou alcényl éthers ou esters sulfates, leurs dérivés et leurs sels, on utilise de préférence ceux pour lesquels le radical alkyle ou alcényle comporte au moins 12 atomes de carbone.
   Parmi les alkyl ou alcényl éthers sulfates, on utilise de préférence les sels d'alkyl éther sulfate et notamment le lauryléther sulfate de sodium. Parmi les alkyl ou alcényl esters sulfates, on peut citer par exemple les esters de l'acide iséthionique ainsi que ses sels et notamment le cocoyl iséthionate de sodium commercialisé sous la dénomination de « Geropon AC 78 » par la Société Rhône Poulenc.
(4) Parmi les alkyl ou alcényl éthers ou esters gras polyoxyéthylénés, on utilise de préférence ceux pour lesquels le radical alkyle ou alcényle comporte au moins 12 atomes de carbone. Ceux particulièrement préférés ont au moins 20 motifs d'oxyde d'éthylène tels que par exemple le PEG-20 stéarate, le laureth-23, l'oleth-20 et le PEG-25 phytostérol.
(5) Parmi les acides alkyl ou alcényl carboxyliques polyoxyéthylénés et leurs sels, on utilise de préférence ceux comportant au moins 10 motifs d'oxyde d'éthylène tels que par exemple l'acide laureth-10 carboxylique et l'acide oleth-10 carboxylique.
(6) Parmi les N-alkyl ou alcényl bétaïnes, on utilise de préférence celles pour lesquelles le radical alkyle ou alcényle comporte au moins 12 atomes de carbone telles que par exemple la lauryl-amidopropyl bétaïne et l'oléyl-amidopropyl bétaïne.
(7) Parmi les alkyl ou alcényl triméthylammonium et leurs sels, on utilise de préférence ceux pour lesquels le radical alkyle ou alcényle comporte au moins 12 atomes de carbone. Comme sels, on utilise de préférence les bromures et chlorures tels que le chlorure de cocoyltriméthylammonium et le bromure de cétyltriméthylammonium.

Lorsque le composé (i) est un dérivé N-2-alcoxycarbonyle de N-méthyl glucamine de formule (I), on utilise de préférence comme agent dispersant et stabilisant (ii), le polyglycéryl-3 hydroxylauryléther, le lauryléthersulfate de sodium ou le bromure de cétyltriméthylammonium.

Selon un second mode de réalisation de l'invention, les particules de gel cubique sont formées par un mélange d'au moins deux composés amphiphiles, l'un des composés amphiphiles étant susceptible de former en présence d'eau une phase lamellaire et l'autre étant susceptible de former en présence d'eau une phase hexagonale inverse.

Le mélange des deux composés amphiphiles formant les particules de gel cubique est caractérisé par le fait qu'aucun des deux composés amphiphiles ne peut conduire par lui-même à une phase cubique lorsqu'il est mis en présence d'eau et que seul leur mélange conduit à une telle phase, et que, par ailleurs, l'un des composés amphiphiles est susceptible de former en présence d'eau une phase lamellaire, tandis que l'autre composé amphiphile est susceptible de former en présence d'eau une phase hexagonale inverse.

On entend par phase lamellaire (phase D selon EKWALL), une phase cristal liquide à symétrie plane, comprenant plusieurs bicouches d'amphiphile disposées en parallèle et séparées par un milieu liquide qui est généralement de l'eau.

On entend par phase hexagonale inverse (phase F selon EKWALL), une phase cristal liquide correspondant à une arrangement hexagonal de cylindres parallèles remplis d'un milieu liquide qui est généralement de l'eau, séparés par un environnement hydrocarboné correspondant aux chaînes grasses de l'amphiphile.

Une définition plus précise de ces dénominations est donnée dans Ekwall (1968), Adv . Liq. Cryst. (Brown G.H., Ed.), Chap. 1, 14. Chacune de ces phases possède une texture caractéristique au microscope en lumière polarisée, dont on pourra trouver une description plus précise dans Rosevear (1968), JSCC, 19, 581. et dans Lachampt et Vila (1969), Revue Française des Corps Gras, n°2, 87-111.

De manière préférée, le composé amphiphile susceptible de former une phase lamellaire est choisi parmi les monoesters de diglycérol, tels que l'isostéarate de diglycérol (Solvay) et le mono-oléate de diglycérol (RYLO PG 29^{R} commercialisé par la société Danisco), seuls ou en mélange.

Le composé amphiphile susceptible de former une phase hexagonale inverse est de préférence choisi parmi les mono-, di- ou tri-esters de diglycérol, les carbamates d'aminopolyols et leurs mélanges. Comme mono-, di- ou tri-esters de diglycérol, on peut citer par exemple le décyl-2 tétradécanoate de diglycérol et le di/tri-oléate de diglycérol (TSED 396^{R} commercialisé par la société Danisco). Comme carbamates d'aminopolyols, on peut citer entre autres le 3-N-(2-décyltétradécyloxycarbonyl)-amino-1,2-propanediol et le N-2-dodécylhexadécyloxycarbonyl-N-méthyl-D-glucamine. Ces carbamates d'aminopolyols sont décrits dans le document EP-A-666251 cité ici pour référence.

De préférence, le mélange des deux types de composés amphiphiles est constitué de 10 à 90 % en poids et mieux 15 à 85 % en poids d'au moins un composé amphiphile susceptible de former une phase lamellaire, et de 10 à 90 % en poids et mieux de 15 à 85 % en poids d'au moins un composé amphiphile susceptible de former une phase hexagonale inverse, par rapport au poids total du mélange.

Le rapport entre les deux types de composés amphiphiles dépend des composés utilisés et l'homme du métier saura déterminer la quantité de chaque type de composé à utiliser afin d'obtenir des particules de gels cubiques.

Plus précisément, les mélanges constituant les particules de gel cubique des compositions de l'invention sont de préférence réalisés à l'aide des associations suivantes :
- 55 à 75 % en poids d'isostéarate de diglycérol et 25 à 45 % en poids de décyl-2 tétradécanoate de diglycérol ;
- 30 à 65 % en poids d'isostéarate de diglycérol et 35 à 70 % en poids de di/tri-oléate de diglycérol ;
- 75 à 85 % en poids d'isostéarate de diglycérol et 15 à 25 % en poids de 3-N-(2-décyl-tétradécyloxycarbonyl)-amino-1,2-propanediol ;
- 55 à 75 % en poids d'isostéarate de diglycérol et 25 à 45 % en poids de N-2-dodécylhexadécyl-oxycarbonyl-N-méthyl-D-glucamine ;
- 15 à 50 % en poids de mono-oléate de diglycérol et 50 à 85 % en poids de di/tri-oléate de diglycérol.

Dans ce second mode de réalisation, le mélange des composés constituant les particules de gel cubique est de préférence réalisé en dispersion aqueuse, et de préférence en présence d'au moins un agent dispersant et stabilisant, celui-ci pouvant être en particulier choisi parmi les composés (ii) indiqués ci-dessus pour le premier mode de réalisation des particules de gel cubique. Quand il est présent, l'agent dispersant et stabilisant est utilisé en une quantité allant par exemple d'environ 0,1 à 3 % en poids par rapport au poids total de la dispersion.

Dans les deux modes de réalisation des particules de gel cubique, décrits ci-dessus, on peut ajouter dans la dispersion aqueuse contenant ces particules, un lipide amphiphile ionique non hydrosoluble, de préférence en une quantité allant de 0,0005 % à 5 % en poids et mieux de 0,001 % à 2 % en poids par rapport au poids total de la dispersion. Parmi les lipides amphiphiles ioniques non hydrosolubles, on peut notamment citer :
(i) les phospholipides tels que les phospholipides naturels comme la lécithine de soja ou d'oeuf, les phospholipides modifiés par voie chimique ou enzymatique comme la lécithine hydrogénée ou le sel de sodium de l'acide phosphatidique, et les phospholipides de synthèse comme la dipalmitoylphosphatidylcholine,
(ii) les esters phosphoriques d'acide gras et leurs sels, notamment de sodium et de potassium, tels que le monocétyl phosphate commercialisé sous la dénomination de "Monafax 160/" par la Société Mona, ainsi que le dimyristyl phosphate commercialisé sous la dénomination de « Mexoryl SY » par la Société Chimex,
(iii) les dérivés N-acylés de l'acide glutamique et leurs sels, tels que le stéaroylglutamate de monosodium commercialisé sous la dénomination de « Acylglutamate HS 11 » par la Société Ajinomoto, et le mélange cocoyl-alcoyl C14-C20 glutamate de monosodium, le radical alcoyle C14-C20 dérivant du suif hydrogéné, commercialisé sous la dénomination de « Acylglutamate GS 11 » par la Société Ajinomoto,
(iv) le cétylsulfate de sodium commercialisé sous la dénomination de « Nikkol SCS » par la Société Nikko Chemicals,
(v) le cocoyl monoglycéride sulfate de sodium commercialisé sous la dénomination de « Nikkol SGC 80 N » par la Société Nikko Chemicals, et
(vi) les dérivés d'ammonium quaternaire non hydrosolubles tels que le chlorure de béhényltriméthylammonium, le chlorure de dilauryldiméthylammonium, le chlorure de distéaryl-diméthylammonium, le méthylsulfate de 4,5-dihydro 1-méthyl 2-alcoyl C14-C20 1-(2-alcoyl C14-C20 aminoéthyl)imidazolium, les radicaux alcoyles C14-C20 dérivant du suif hydrogéné, commercialisé sous la dénomination de « Rewoquat W75H » par la Société Rewo Chemische, le méthylsulfate de dialkylhydroxyéthylméthylammonium dont les radicaux alkyles dérivent du suif, hydrogéné ou non, commercialisé sous la dénomination de « Stepanquat VP 85 » par la Société Stepan et le « Quaternium-82 » commercialisé par la Société Seppic sous la dénomination de « Amonyl DM ».

L'incorporation de ces lipides amphiphiles ioniques non hydrosolubles confère aux particules de gel cubique une charge superficielle entraînant une répulsion électrostatique des particules entre elles.

Les particules de gel cubique, telles que définies précédemment, ont une taille qui peut être modulée par la nature et la concentration des composés les constituant. Généralement, ces particules ont une taille moyenne en nombre, mesurée à l'aide d'un granulomètre laser BI 90 de la Société Brookhaven Instruments Corporation, d'environ 0,05 µm à environ 1 µm, et de préférence inférieure ou égale à 0,5 µm.

Il est en outre possible d'incorporer dans les particules de gel cubique, divers types de composés actifs. En particulier, lesdites particules peuvent contenir un principe actif hydrophile ou lipophile. Bien entendu, grâce à la structure particulière des particules de gel cubique, il est possible d'incorporer dans celles-ci à la fois des principes actifs hydrophiles et lipophiles, même si ceux-ci présentent une certaine incompatibilité.

Les compositions utilisées selon l'invention peuvent constituer notamment des compositions cosmétiques et dermatologiques. Elles contiennent pour une telle application, un milieu physiologiquement acceptable. On entend ici par « milieu physiologiquement acceptable », un milieu compatible avec la peau, les lèvres, le cuir chevelu, les cils, les yeux et/ou les cheveux. Ce milieu physiologiquement acceptable peut être plus particulièrement constitué d'eau et éventuellement d'un solvant organique physiologiquement acceptable choisi par exemple parmi les alcools inférieurs comportant de 1 à 4 atomes de carbone comme l'éthanol, l'isopropanol, le propanol, le butanol ; les polyéthylène glycols ayant de 6 à 80 oxydes d'éthylène ; les polyols comme le propylène glycol, l'isoprène glycol, le butylène glycol, la glycérine, le sorbitol. Le milieu physiologiquement acceptable de la composition selon l'invention a un pH compatible avec la peau, et de préférence allant de 3 à 8 et mieux de 5 à 7.

Selon un mode préférée de réalisation, les compositions utilisées dans la présente invention comportent en outre une phase huileuse, apportant notamment du confort et de la douceur lors de l'application sur la peau. La quantité de phase huileuse peut aller par exemple de 2 à 40 % en poids et de préférence de 5 à 25 % en poids par rapport au poids total de la composition, le reste de la composition étant constitué de la phase aqueuse contenant le phytantriol ou constituée par les particules de gel cubique contenant le phytantriol ou contenant la dispersion aqueuse des particules de gel cubique contenant le phytantriol. Quand le phytantriol est dans des particules de gel cubique, le rapport en poids des composés amphiphiles constituant les particules de la phase cubique et de la phase huileuse va de préférence de 0,02/1 à 1/1, et mieux de 0,05/1 à 0,5/1.

Comme huiles utilisables dans l'invention, on peut citer les huiles minérales (huile de vaseline), les huiles végétales (fraction liquide du beurre de karité, huile de tournesol, huile d'amande d'abricot), les huiles animales (perhydrosqualène), les huiles de synthèse (polyisobutène hydrogénée, néopentanoate d'isostéaryle, myristate d'isopropyle), les huiles de silicone non volatiles ou volatiles (cyclométhicones telles que cyclopentasiloxane et cyclohexasiloxane) et les huiles fluorées (perfluoropolyéthers). On peut aussi utiliser, comme matières grasses, des alcools gras, des acides gras, des cires. La phase huileuse de l'émulsion peut contenir aussi des gommes telles que les gommes de silicone, des résines et des cires.

La composition contenant une phase huileuse peut être sous forme d'une émulsion eau-dans-huile (E/H) ou huile-dans-eau (H/E). Selon un mode préféré de réalisation, elle est sous forme d'une émulsion huile-dans-eau.

De façon connue, les compositions de l'invention peuvent contenir également des adjuvants habituels dans les domaines cosmétique ou dermatologique, tels que les gélifiants hydrophiles ou lipophiles, les actifs hydrophiles ou lipophiles, les conservateurs, les antioxydants, les solvants, les parfums, les charges, les filtres, les bactéricides, les absorbeurs d'odeur, les matières colorantes, les sels. Les quantités de ces différents adjuvants sont celles classiquement utilisées dans le domaine considéré, et par exemple de 0,01 à 10 % du poids total de la composition. Ces adjuvants, selon leur nature, peuvent être introduits dans la phase grasse, dans la phase aqueuse et/ou dans des sphérules lipidiques.

Comme actifs, la composition peut notamment contenir d'autres actifs anti-pollution tels que les sphingolipides (voir EP-A-0 577 718), des filtres tels que l'octocrylène et le butylméthoxydibenzoylméthane, des hydratants tels que les polyols et notamment la glycérine.

Comme gélifiants, on peut citer par exemple les dérivés de cellulose tels que l'hydroxyéthylcellulose et les alkylhydroxyéthylcelluloses telles que la cétylhydroxyéthylcellulose ; les dérivés d'algues tels que le satiagum ; les gommes naturelles telles que l'adragante ou la gomme guar ; les polymères synthétiques tels que les polymères ou copolymères carboxyvinyliques et en particulier ceux commercialisés sous les dénominations de Carbopol^{R} par la société Goodrich ou de Synthalen^{R} par la société 3V SA. La proportion en agent gélifiant va de préférence de 0,1 à 2 % du poids total de la composition.

Les compositions utilisées selon l'invention peuvent être plus ou moins fluides et avoir l'aspect d'une crème blanche ou colorée, d'une pommade, d'un lait, d'une lotion, d'un sérum, d'une pâte, d'une mousse. Elles peuvent être éventuellement appliquées sur la peau sous forme d'aérosol. Elles peuvent aussi se présenter sous forme solide, et par exemple sous forme de stick.

De préférence, les compositions utilisées selon l'invention sont obtenues selon un procédé de préparation comprenant au moins deux étapes. La première étape consiste généralement à préparer une dispersion aqueuse de particules de gel cubique, telles que définies précédemment, par fragmentation, à l'aide d'un homogénéiseur, des composés tels que définis précédemment et d'eau, en présence éventuellement de lipides amphiphiles ioniques non hydrosolubles et/ou de principes actifs hydrophiles et/ou lipophiles et/ou d'un agent dispersant et stabilisant tels que définis précédemment. L'homogénéiseur peut être du type rotor-stator à fort gradient de cisaillement comme le Virtis^{R} ou le Heidolph Diax 600^{R} ou un homogénéiseur haute pression fonctionnant entre 200 et 1.800 bars environ (20 à 180 MPa).

Il est bien entendu possible d'introduire, à ce stade de la préparation de la dispersion aqueuse des particules de gel cubique, divers additifs et/ou principes actifs dans la phase aqueuse. Après la formation des particules de gel cubique, l'agent dispersant et stabilisant se trouve, généralement, à l'extérieur desdites particules.

La deuxième étape consiste généralement ensuite à ajouter à ladite dispersion obtenue une phase huileuse contenant éventuellement certains additifs et/ou principes actifs lipophiles et à soumettre le mélange à une agitation mécanique qui peut être notamment réalisée à l'aide d'un homogénéiseur du même type que ceux définis ci-dessus.

Divers additifs et/ou principes actifs peuvent être également introduits à ce stade de la préparation. Par ailleurs, lorsque l'on souhaite préparer une dispersion gélifiée, dans une troisième étape, on ajoute généralement au mélange obtenu après la seconde étape, une solution aqueuse contenant un agent gélifiant.

Les compositions utilisées selon l'invention peuvent notamment constituer un produit de soin et/ou de maquillage. Elles peuvent être utilisées notamment pour protéger l'organisme contre les effets de la pollution et/ou pour améliorer la respiration cellulaire et/ou diminuer la desquamation et/ou pour éviter de ternir ou de salir les matières kératiniques, et notamment la peau.

Ainsi, un autre objet de l'invention consiste en un procédé de traitement cosmétique en vue de protéger une matière kératinique contre les effets de la pollution, consistant à appliquer sur la matière kératinique, une composition contenant dans un milieu physiologiquement acceptable, une quantité efficace de particules de gel cubique.

L'invention a aussi pour objet un procédé de traitement cosmétique en vue d'améliorer la respiration cellulaire et/ou diminuer la desquamation et/ou pour éviter de ternir et/ou de salir la matière kératinique, consistant à appliquer sur la matière kératinique, une composition contenant dans un milieu physiologiquement acceptable, une quantité efficace de particules de gel cubique.

Les exemples qui suivent servent à illustrer l'invention sans toutefois présenter un caractère limitatif. Les noms sont, selon le cas, en noms chimiques ou noms CTFA (International Cosmetic Ingredient Dictionary and Handbook) et les quantités en pourcentage en poids sauf mention contraire.

### Exemple 1 :

### Phase A :

- Phytantriol 3,92 %
- Cetyl phosphate (commercialisé sous la dénomination « Arlatone MAP160 » par la Société Uniquema) 0,08 %
- Eau 1,6 %

### Phase B :

- Polysorbate 40 (commercialisé sous la dénomination « Montanox 40 DF » par la Société Seppic) (agent dispersant) 1 %
- Triéthanolamine 0,04 %
- Eau 55,96 %
- Conservateur 0,3 %

### Phase C :

- Polyisobutène hydrogéné 7,8 %
- Cyclohexasiloxane 11,6 %
- Néopentanoate d'isostearyle 2,6 %
- parfum 0,1 %

### Phase D :

- Cetyl hydroxyethylcellulose (commercialisé sous la dénomination « Natrosol Plus Grade 330CS » par la Société Hercules) 1 %
- Eau 14 %

### Mode opératoire :

### Première étape :

On mélange à température ambiante les composés de la phase A. A ce mélange on ajoute à température ambiante la phase B. Le mélange est ensuite dispersé et homogénéisé à température ambiante à l'aide d'un homogénéiseur de type « UltraTurrax T50 » muni d'une tête de dispersion 45F, à 10000 rpm pendant 30 minutes.

### Deuxième étape :

A la dispersion aqueuse de particules de gel cubique, obtenue ci-dessus, on ajoute le mélange huileux de la phase C. Le mélange est alors homogénéisé à température ambiante à l'aide d'un homogénéiseur haute pression par 4 passages à 600 bar.

### Troisième étape :

La préparation obtenue à la deuxième étape est gélifiée à l'aide du mélange de la phase D. On homogénéise alors le mélange à température ambiante à l'aide d'un homogénéiseur à pale pendant 30 minutes. On obtient une crème homogène et stable qui s'applique facilement sur la peau et protège de la pollution.

### Exemple A (comparatif) : Emulsion huile dans eau

- Polyisobutène hydrogéné 7,8 %
- Cyclohexasiloxane 11,6 %
- Néopentanoate d'isostearyle 2,6 %
- Conservateurs 0,5 %
- Gomme de xanthane 0,6 %
- Polyacrylamide */* C13-14 isoparaffin */* laureth-7 (Sepigel 305 commercialisé par la société SEPPIC) 2 %
- Diméthicone copolyol (DC2-5695 de la société Dow Corning) 3 %
- Cyclohexasiloxane 11,7 %
- Glycérine 3 %
- Eau qsp 100 %

### Exemple B (comparatif) : Emulsion eau dans huile

- Polyisobutène hydrogéné 7,8 %
- Cyclohexasiloxane 11,7 %
- Néopentanoate d'isostearyle 2,6 %
- Chlorure de sodium 0,6 %
- Cétyldiméthicone copolyol (Abil EM90 de la société Goldschmidt) 3 %
- Glycérine 3 %
- Eau qsp 100 %

### Test pour la mise en évidence in vitro de l'effet protecteur des particules de gel cubique

### Efficacité anti-pollution sur peau reconstruite

Les compositions de l'exemple 1 et des exemples comparatifs A et B ont été appliquées (2 mg/cm²) à la surface des épidermes de peau reconstruite et laissées à leur contact pendant 30 minutes à température ambiante. Des particules radio-marquées au carbone 14 ont été ensuite déposées sur les épidermes et laissées à leur contact pendant 2 heures dans le milieu de maintenance habituelles des épidermes. Puis les épidermes ont été retirés de leur milieu de maintenance et lavés plusieurs fois au tampon PBS (Phosphate Buffer Solution). Les lavages permettent de débarrasser les épidermes des particules faiblement adsorbées sans éliminer la composition initialement appliquée. Les taux de particules radio-marquées résiduels ont été ensuite évalués par la mesure de la radioactivité du carbone 14 additionné aux particules. Le tableau ci-dessous donne les résultats en pourcentage de particules résiduelles par rapport à la quantité de particules déposées

| | % par rapport à la quantité de particules déposées |
|---|---|
| Zone non traitée | 36,2 ± 2,83% |
| Zone traitée avec composition de l'exemple 1 | 3,3 ± 0,66 |
| Zone traitée avec émulsion de l'exemple A (comparatif) | 11,3 ± 0,65 |
| Zone traitée avec émulsion de l'exemple B (comparatif) | 11,2 ±2,10 |

Ces résultats montrent que les compositions contenant les particules de gel cubique, utilisées selon l'invention permettent une meilleure protection de la peau contre les particules polluantes que les émulsions classiques, en limitant la pénétration des particules polluantes externes.

### Exemple 2 : Fluide (émulsion H/E)

Selon le même mode opératoire que pour l'exemple 1, on a préparé un fluide de jour sous forme d'une dispersion par mélange des parties suivantes :

### Phase A :

- Phytantriol 2,97 %
- Sel monosodique de l'acide N-stéaroylglutamique (commercialisé sous la dénomination Acylglutamate HS-11 par la Société Ajinomoto) 0,03 %
- Eau 1,25 %

### Phase B :

- Polysorbate 40 (commercialisé sous la dénomination « Montanox 40 DF » par la Société Seppic) (agent dispersant) 0,75 %
- Glycérine 4 %
- Propylène glycol 4 %
- Eau 55,8 %
- Conservateur 0,2 %

### Phase C :

- Octocrylène 8,4 %
- Butyl methoxydibenzoylmethane 3,6 %
- Dimethicone (DC200 Fluid 1.5 cSt) 4 %
- Néopentanoate d'isostearyle 2 %
- Myristate d'isopropyle 2 %

### Phase D :

- Conservateur 1 %
- Eau 10 %

On obtient une composition fluide qui peut être appliquée sous forme d'un spray et qui permet une bonne protection de la peau.

### Exemple 3 : lait (émulsion H/E)

Selon le même mode opératoire que dans l'exemple 1, on a préparé un fluide de jour sous forme d'une dispersion par mélange des parties suivantes :

### Phase A :

- Isostéarate de diglycérol (Solvay) 1,8 %
- Cetyl phosphate (commercialisé sous la dénomination « Arlatone MAP160 » par la Société Uniqema) 0,03 %
- Decyl-2 tétradécanoate de diglycérol 1,2 %
- Eau 1,25 %

### Phase B :

- Triéthanolamine 0,01 %
- Glycérine 3 %
- Eau 63 %
- Conservateur 0,3 %

### Phase C :

- Perhydrosqualène 5 %
- Heptanoate de stéaryle / octanoate de stéaryle 4 %
- Huile d'amande d'abricot 5 %
- Cyclohexasiloxane 2 %
- Conservateur 0,1 %

### Phase D :

- Cetyl hydroxyethylcellulose (commercialisé sous la dénomination « Polysurf 67» par la Société Hercules) 0,5 %
- Eau 12,81 %

On obtient un lait agréable à appliquer sur la peau et constituant une bonne protection contre les polluants.

### Exemple 4 : Crème de jour (émulsion H/E)

Selon le même mode opératoire que pour l'exemple 1, on a préparé une crème de jour par mélange des parties suivantes :

### Phase A :

- Isostéarate de diglycérol (Solvay) 1,2 %
- Di/tri-oléate de diglycérol (commercialisé sous la dénomination « TS-ED 396 » par la Société Danisco) 1,8 %
- Eau 1,25 %

### Phase B :

- Polysorbate 40 (commercialisé sous la dénomination « Montanox 40 DF » par la Société Seppic) 1 %
- Glycérine 3 %
- Eau 60,35 %
- Conservateur 0,3 %

### Phase C :

- Perhydrosqualène 4 %
- Huile d'amande d'abricot 7 %
- Cyclopentasiloxane 5 %
- Conservateur 0,1 %

### Phase D :

- Cetyl hydroxyethylcellulose (commercialisé sous la dénomination « Natrosol Plus Grade 330CS » par la Société Hercules) 1 %
- Eau 14 %

On obtient une crème de jour, confortable et apte à protéger la peau de la pollution de manière efficace.

## Revendications

1. Utilisation cosmétique de particules de gel cubique comme agent anti-pollution dans une composition pour application topique sur les matières kératiniques.

2. Utilisation de particules de gel cubique pour la préparation d'une composition à application topique destinée à protéger la matière kératinique contre les effets nocifs de la pollution.

3. Utilisation selon la revendication 1 ou 2, **caractérisée par le fait que** les particules de gel cubique sont en dispersion aqueuse.

4. Utilisation selon la revendication précédente, **caractérisée par le fait que** les particules de gel cubique sont formées par un mélange comprenant :
(i) de 0,1 à 15 % en poids par rapport au poids total de la composition, d'au moins un composé choisi parmi le 3,7,11,15-tétraméthyl-1,2,3-hexadecanetriol ou phytantriol, les dérivés N-2-alcoxycarbonyles de N-méthylglucamine, et les monoglycérides d'acide gras insaturé, et
(ii) de 0,05 à 3 % en poids par rapport au poids total de la composition, d'au moins un agent dispersant et stabilisant, ledit agent étant choisi parmi les agents tensioactifs hydrosolubles à température ambiante, contenant une chaîne grasse, saturée ou insaturée, linéaire ou ramifiée, ayant de 8 à 22 atomes de carbone.

5. Utilisation selon la revendication 4, **caractérisée par le fait que** la proportion pondérale relative en composé (i) par rapport au poids dudit agent dispersant et stabilisant (ii) va de 2 à 200.

6. Utilisation selon la revendication 4 ou 5, **caractérisée par le fait que** ledit dérivé N-2-alcoxycarbonyle de N-méthylglucamine répond à la formule (I) suivante 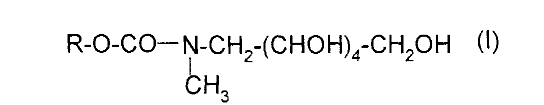 dans laquelle R représente un radical alkyle ramifié comportant de 6 à 18 atomes de carbone.

7. Utilisation selon l'une quelconque des revendications 4 à 6, **caractérisée par le fait que** ledit dérivé N-2-alcoxycarbonyle de N-méthyl-glucamine est choisi parmi la N-2-hexyldécyloxycarbonyl-N-méthyl-glucamine, la N-2-éthylhexyloxycarbonyl-N-méthyl-glucamine, la N-2-butyloctyloxycarbonyl-N-méthyl-glucamine et leurs mélanges.

8. Utilisation selon l'une quelconque des revendications 4 à 7, **caractérisée par le fait que** les particules de gel cubique contiennent comme composé (i), un mélange constitué de 1 à 40 % en poids de phytantriol par rapport au poids du mélange et de 60 à 99 % en poids de dérivé N-2-alcoxycarbonyle de N-méthyl-glucamine par rapport au poids du mélange.

9. Utilisation selon la revendication 4, **caractérisée par le fait que** ledit monoglycéride d'acide gras insaturé est choisi parmi le monooléate de glycéryle et le monolinoléate de glycéryle.

10. Utilisation selon la revendication 4, **caractérisée par le fait que** les particules de gel cubique contiennent comme composé (i) un mélange constitué de 1 à 50 % en poids de phytantriol par rapport au poids du mélange, et de 50 à 99 % en poids de monoglycéride d'acide gras insaturé par rapport au poids du mélange.

11. Utilisation selon l'une quelconque des revendications 4 à 10, **caractérisée par le fait que** ledit agent dispersant et stabilisant est choisi parmi :
(1) les alkyl ou alcényl éthers ou esters de polyol,
(2) les amino-acides N-acylés et leurs dérivés et les peptides N-acylés par un radical alkyle ou alcényle, et leurs sels,
(3) les alkyl ou alcényl éthers ou esters sulfates, leurs dérivés et leurs sels,
(4) les alkyl ou alcényl éthers ou esters gras polyoxyéthylénés,
(5) les acides alkyl ou alcényl carboxyliques polyoxyéthylénés et leurs sels,
(6) les N-alkyl ou alcényl bétaïnes,
(7) les alkyl ou alcényl triméthylammonium et leurs sels, et
(8) leurs mélanges.

12. Utilisation selon l'une quelconque des revendications 1 à 3, **caractérisée par le fait que** les particules de gel cubique sont formées par un mélange d'au moins deux composés amphiphiles, l'un des composés amphiphiles étant susceptible de former en présence d'eau une phase lamellaire et l'autre étant susceptible de former en présence d'eau une phase hexagonale inverse.

13. Utilisation selon la revendication 12, **caractérisée par le fait que** le composé amphiphile susceptible de former une phase lamellaire est choisi parmi les monoesters de diglycérol.

14. Utilisation selon la revendication 12 ou 13, **caractérisée par le fait que** le composé amphiphile susceptible de former une phase hexagonale inverse est choisi parmi les mono-, di- ou tri-esters de diglycérol, les carbamates d'aminopolyols et leurs mélanges.

15. Utilisation selon l'une quelconque des revendications 12 à 14, **caractérisée par le fait que** le composé amphiphile susceptible de former une phase lamellaire est choisi parmi l'isostéarate de diglycérol, le mono-oléate de diglycérol et leurs mélanges.

16. Utilisation selon l'une quelconque des revendications 12 à 15, **caractérisée par le fait que** le composé amphiphile susceptible de former une phase hexagonale inverse est choisi parmi le décyl-2 tétradécanoate de diglycerol, le di/tri-oléate de diglycérol, le 3-N-(2-décyl-tétradécyloxycarbonyl)-amino-1,2-propanediol et le N-2-dodécylhexadécyl-oxycarbonyl-N-méthyl-D-glucamine, et leurs mélanges.

17. Utilisation selon l'une quelconque des revendications 12 à 16, **caractérisée par le fait que** le mélange des deux composés amphiphiles est constitué de 10 à 90 % en poids du composé amphiphile susceptible de former une phase lamellaire et de 10 à 90 % en poids du composé amphiphile susceptible de former une phase hexagonale inverse, par rapport au poids total du mélange.

18. Utilisation selon l'une quelconque des revendications 12 à 17, **caractérisée par le fait que** le mélange des deux composés amphiphiles est choisi parmi les mélanges suivants :
- 55 à 75 % en poids d'isostéarate de diglycérol et 25 à 45 % en poids de décyl-2 tétradécanoate de diglycérol ;
- 30 à 65 % en poids d'isostéarate de diglycérol et 35 à 70 % en poids de di/tri-oléate de diglycérol;
- 75 à 85 % en poids d'isostéarate de diglycérol et 15 à 25 % en poids de 3-N-(2-décyl-tétradécyloxycarbonyl)-amino-1,2-propanediol ;
- 55 à 75 % en poids d'isostéarate de diglycérol et 25 à 45 % en poids de N-2-dodécylhexadécyl-oxycarbonyl-N-méthyl-D-glucamine ;
- 15 à 50 % en poids de mono-oléate de diglycérol et 50 à 85 % en poids de di/tri-oléate de diglycérol.

19. Utilisation selon l'une quelconque des revendications précédentes, **caractérisée par le fait que** les particules de gel cubique ont une taille allant de 0,05 µm à 1 µm.

20. Utilisation selon l'une quelconque des revendications 3 à 19, **caractérisée par le fait que** la dispersion de particules de gel cubique comprend en outre au moins un lipide amphiphile ionique non hydrosoluble.

21. Utilisation selon la revendication précédente, **caractérisée par le fait que** ledit lipide amphiphile ionique non hydrosoluble est choisi parmi :
(i) les phospholipides,
(ii) les esters phosphoriques d'acide gras,
(iii) les dérivés N-acylés de l'acide glutamique non hydrosolubles et leurs sels,
(iv) le cétylsulfate de sodium,
(v) le cocoyl monoglycéride sulfate de sodium, et
(vi) les dérivés d'ammonium quaternaire non hydrosolubles.

22. Utilisation selon l'une quelconque des revendications précédentes, **caractérisée par le fait que** les particules comprennent au moins un principe actif hydrophile et/ou lipophile.

23. Utilisation selon l'une quelconque des revendications précédentes, **caractérisée par le fait que** les particules de gel cubique sont présentes en une quantité allant de 0,1 à 20 % en poids par rapport au poids total de la composition.

24. Procédé de traitement cosmétique en vue de protéger une matière kératinique contre les effets de la pollution, consistant à appliquer sur la matière kératinique, une composition contenant dans un milieu physiologiquement acceptable, une quantité efficace de particules de gel cubique.

25. Procédé de traitement cosmétique en vue d'améliorer la respiration cellulaire et/ou diminuer la desquamation et/ou pour éviter de ternir et/ou de salir une matière kératinique, consistant à appliquer sur la matière kératinique, une composition contenant dans un milieu physiologiquement acceptable, une quantité efficace de particules de gel cubique.

26. Procédé selon l'une quelconque des revendications 24 ou 25, **caractérisée par le fait que** la matière kératinique est la peau.
